# EUROPEAN PATENT SPECIFICATION

(11) **EP 2 393 437 B1**
(45) Date of publication and mention of the grant of the patent: **19.04.2017**
(21) Application number: 10710426.7
(22) Date of filing: 01.02.2010
(51) Int. Cl.: A61B 17/22, A61B 17/00

(54) **MEDICAL DEVICE**
MEDIZINISCHE VORRICHTUNG
DISPOSITIF MÉDICAL

(30) Priority: 30.01.2009 GB 0901610; 30.01.2009 GB 0901614; 30.01.2009 GB 0901612
(43) Date of publication of application: 14.12.2011
(73) Proprietor: Cook Medical Technologies LLC, Bloomington, IN 47404 (US)
(72) Inventor: LEBET, Alain, 1006 Lausanne (CH)
(74) Representative: Garratt, Peter Douglas
(86) International application number: PCT/IB2010/000308
(87) International publication number: WO 2010/086741

(56) References cited:
- US-A- 5 462 522
- US-A1- 2003 144 680
- US-A1- 2005 261 715
- US-A1- 2008 319 376
- US-B1- 6 425 883
- US-B2- 6 695 782

## Description

This invention relates to a medical device for the safe and efficient removal of urinary tract stones.

Urinary tract stones are solid concretions formed from minerals present in urine. Small stones can pass from the body in urine without notice, however, larger stones can become lodged in the ureter, kidneys or bladder causing discomfort and/or pain.

There are many different techniques used to treat urinary tract stones ranging from non-invasive techniques, such as extracorporeal shock wave lithotripsy (ESWL), to invasive techniques such as surgery, basket extraction, laser fragmentation, mechanical shock-wave fragmentation or ultrasonic fragmentation. If fragmentation is used, the fragments of the stone may be allowed to pass from the body in the urine alternatively the fragments may be extracted using basket extraction or suction.

Medical devices comprising ultrasonic probes (e.g. probes transmitting energy in the range of from 20 kHz to 200 kHz) have a variety of applications, including the fragmentation and/or wear of urinary tract stones or the removal of occlusions in blood vessels. Ultrasonic fragmentation commonly uses a piezoelectric motor to generate ultrasound from a high frequency electrical voltage. The piezoelectric motor is usually located in a handpiece and the vibrations resulting from the ultrasound are usually transmitted to a probe that is inserted via an endoscope into the body. This allows the tip of the probe to come into direct contact with a urinary stone, such that the vibrations cause the stone to fragment or wear.

Often a urinary stone will fragment or wear after only a few seconds of vibrations being applied, however, longer periods of application of vibrations may be required to fragment or wear some stones and/or repetitive application of vibrations may be necessary to result in sufficiently small fragments. The longer the period of time the probe is used for the more the vibrations will contribute to an increase in the temperature of the local environment of the probe. Since the probe is in an inserted location within a patient, overheating is a serious problem. If the probe is allowed to reach excessively high temperatures the patient's body tissue can be damaged. US 2008/319376 A1 relates to an ultrasound catheter with fluid delivery lumens, fluid evacuation lumens and a light source which is used for the treatment of intracerebral hemorrhages.

The present invention offers an improved device for the removal of urinary tract stones. The invention is defined in claim 1. Preferred embodiments are defined in the dependent claims.

In accordance with a first aspect of the present disclosure there is provided a medical device comprising a body and a hollow probe, ultrasound generation means and suction monitoring means, wherein, in use, suction is applied through the hollow probe, and wherein the ultrasound generation means is adjusted in response to a suction flow rate detected by the suction monitoring means. Suction may be supplied from a vacuum system within the hospital in which the device is being used, or may be supplied by a separate suction pump.

If the suction flow rate reduces the ultrasound generation means may be activated in response and if, following activation of the ultrasound generation means, the suction flow rate increases the ultrasound generation means may be deactivated in response. The ultrasound generation means may be activated and/or deactivated manually or automatically in response to a signal generated by the suction monitoring means. The signal may be an audible and/or visible alarm to enable a user of the device to manually respond to the suction flow rate detected or the signal may be an electronic signal enabling computer control means to automatically respond to the suction flow rate detected. Of course both manual and automatic control of the ultrasound generation means in response to a suction flow rate detected by the suction monitoring means may be enabled. Additionally the frequency and strength of the ultrasound may be varied manually or automatically in response to the extent of the reduction or increase in the suction flow rate.

The suction flow rate can be reduced by a fragment of a urinary tract stone becoming lodged within the hollow probe or by a stone blocking the tip of the hollow probe because it is too large to enter into the probe. Activating the ultrasound generation means when a blockage is detected allows the hollow probe to be used to fragment or wear the stones and thereby clear the blockage. Continuing to apply suction during a blockage ensures that the stone is held in contact with the probe surface and therefore ensures that the ultrasound vibrations are transmitted efficiently to the stone or stone fragment, resulting in the efficient fragmentation or wear of the stone or stone fragment.

The medical device of the present disclosure may be used on its own to fragment, wear and extract stones or may be used following a separate fragmentation procedure to extract the fragmented stones produced by the previous procedure.

The suction monitoring means may comprise flow sensors to directly monitor the flow rate of the fluids passing through the hollow probe. The suction means may comprise pressure sensors to monitor the flow rate with reference to the pressure of the fluids flowing through the hollow probe. Alternatively, the suction monitoring means may comprise both flow sensors and pressure sensors.

In one embodiment of the present invention, the medical device comprises temperature monitoring means and the ultrasound generation means is adjusted in response to a temperature detected by the temperature monitoring means.

If the temperature monitoring means detects that the hollow probe has reached a high temperature, such as a temperature in excess of 40°C, the ultrasound generation means may be adjusted or deactivated in response. The ultrasound generation means may be activated and/or deactivated manually or automatically in response to a signal generated by the temperature monitoring means. The signal may be an audible and/or visible alarm to enable a user of the device to manually respond to the temperature detected or the signal may be an electronic signal enabling computer control means to automatically respond to the temperature detected. Of course both manual and automatic control of the ultrasound generation means in response to a temperature detected by the temperature monitoring means may be enabled. Additionally the frequency and strength of the ultrasound may be varied manually or automatically in response to the temperature detected by the temperature monitoring means. This ensures that the probe cannot reach temperatures at which damage may be caused to a patient's body tissue.

The temperature monitoring means may comprise at least one temperature sensor. A temperature sensor may be coupled to the hollow probe to directly measure the temperature of the hollow probe. A temperature sensor may also be located within the device so that, in use, it measures the temperature of the fluid flowing through the device. Alternatively, or in addition to the temperature sensors, the temperature monitoring means may comprise means to calculate the temperature of the probe using the amount of energy used by the device.

In one embodiment, the hollow probe is reusable; however, the hollow probe may also be disposable.

The hollow probe will commonly have an external diameter of from 3 mm to 6 mm and/or an internal diameter of from 2 mm to 5 mm. Additionally, the hollow probe may range in length from 200 mm to 400 mm. The hollow probe may comprise at least one of titanium, stainless steel and another biocompatible metal.

Suitable ultrasound generation means will commonly comprise a piezoelectric motor.

In one embodiment the ultrasound generation means may be adjusted to alter the strength and/or the frequency of the ultrasound, this can increase the efficiency of the ultrasound.

Many medical devices use probes to enter a patient's body. Often the probes need to be fixed to the medical device such that they are able to rotate, vibrate or move in some way. Additionally, such probes often need to be disconnected from and reconnected to the device. This may be because the physician wishes to exchange one size of probe for another, because the probes used are reusable and a recently used probe needs to be cleaned and sterilised before re-use or because the probes used are disposable and so the medical device requires a new probe to be fitted for use in each procedure.

Prior art fixation systems commonly comprise screw threads, clips or grips. For example US 6695782 discloses multiple examples of fixation systems for a device that removes occlusions in blood vessels using an ultrasonic probe, in which the probe is reversibly attached to a handpiece using complementary screw threads on the body of the device and the base of the probe or using a collet-type grip.

Some medical devices comprise bodies with end caps, the exchange of a reversibly attached probe with such medical devices has previously required the complete disconnection of the end cap from the body of the medical device, removal of the old probe by withdrawing its whole length through the hole at the head of the end cap, insertion of a new probe by passing its whole length through the same hole in the end cap, and reconnection of the end cap to the body of the device. This is a time consuming and awkward procedure, and enables contamination of a new probe by an old probe due to material being transferred onto the area around the hole in the end cap as the old probe is withdrawn through the hole and then the new probe contacting this material as it is passed through the same hole. Contamination can be particularly problematic if the probe is hollow, as the contamination may lead to blockage of the probe.

In one example of the present disclosure an improved means of fixing a probe to the body of a medical device comprises at least one chuck for reversibly fixing the probe to the body of the device. The at least one chuck may be used to fix any kind of probe to the body of a lithotripsy device, but is particularly suited to fixing the probes of ultrasonic lithotripsy devices, suction lithotripsy devices and mechanical shock-wave fragmentation lithotripsy devices.

The easy exchange of a probe, is particularly helpful for medical devices using hollow probes (e.g. lithotripsy suction probes) as these probes can become blocked (e.g. by a urinary tract stone or stone fragment). Hollow probes tend to be used to suck a mixture of liquid(s) and solid(s) out of the body of a patient. Since the liquid(s) could damage parts of the device, it is preferable that when a hollow probe is fixed to the body of the device a watertight seal is created.

The at least one chuck may be located within an end cap located on the distal end of the body of the device. Exchange of the probe with such a fixation system is simpler than with prior art systems. The process for exchanging the probe only requires the connection between the end cap and the body of the device to be loosened, which loosens the grip of the at least one chuck on the old probe, the old probe can then be removed by disconnecting the base of the probe from the at least one chuck within the cap, a new probe can be inserted by introducing the base of the new probe into the hole at the head of the cap to enable the base of the probe to connect with the at least one chuck within the cap, and tightening the connection between the cap and the body of the device also tightens the grip of the at least one chuck on the new probe. Depending on the shape and configuration of the end cap, the loosening and tightening of the end cap can act directly on the at least one chuck or the loosening and tightening of the end cap can act on a mechanism within the end cap which in turn acts on the at least one chuck.

Alternatively, the probe may pass through the body of the device from the distal end of the body of the device to the proximal end of the body of the device and the at least one chuck is located at the proximal end of the body of the device. The at least one chuck at the proximal end of the body of the device may also be located within an end cap. The process for exchanging a probe using a proximal end cap requires the connection between the end cap and the body of the device to be loosened, removal of the old probe by disconnecting the base of the probe from the at least one chuck within the cap, inserting a new probe by introducing the base of the new probe through the body of the device to enable the base of the probe to connect with the at least one chuck within the cap, and tightening the connection between the cap and the body of the device.

The at least one chuck may be located within an end cap at either the distal end or the proximal end of the device, or in a third example at least one chuck is located within an end cap at the distal end of the device and at least one chuck is located within an end cap at the proximal end of the device. In this example the probe passes through a distal end cap and through the body of the device to the proximal end of the body of the device.

Both the distal end cap and the proximal end cap may be wholly detachable from the body of the device to assist in sterilisation. However, in alternative examples the distal end cap and the proximal end cap may be attached to the body of the device such that they may only be loosened during exchange of the probes, but not removed.

A first ring may be used to help locate the at least one chuck accurately on the body of the device. Such a ring may be located within an end cap between the at least one chuck and the distal or proximal end of the body of the device. The first ring may comprise metal.

A second ring may be used to help create a seal between an end cap and the body of the device when an end cap is connected to the body. Such a ring may be located between an end cap and the body of the device. The second ring may comprise rubber.

The at least one chuck should be made of a hard and flexible material, and should be suited to efficient ultrasound transmission. Commonly the at least one chuck will comprise a metal such as titanium or stainless steel, however, reinforced plastics have also been shown to be suited to ultrasound transmission. Additionally, since the body of the device may come into contact with bodily fluids during use, parts of the body of the device will commonly require sterilisation following each procedure the device is used in. Sterilisation usually involves the parts requiring sterilisation being placed in an autoclave at an elevated temperature (e.g. 134 °C) for a minimum period of time (e.g. 18 mins). Since the at least one chuck may need to be sterilised the use of materials suited to repetitive sterilisation is preferred.

Many medical devices comprise handpieces that a practitioner holds when the device is in use. These handpieces need to be ergonomically designed, easy to manipulate and lightweight. The requirement that handpieces be lightweight is especially important for handpieces that are used in time consuming procedures when the practitioner needs to be able to use the handpiece comfortably and confidently whilst bearing the weight of the handpiece throughout the procedure. In such time consuming procedures, it has been found to be desirable that the weight of the handpiece is minimised.

If a medical device comprising a handpiece requires electrical power during operation, a power supply will be required. Mains electricity could be used, however, connection to a supply of mains electricity requires the use of power cables that may trail across the floor of the operating theatre creating a hazard. Additionally, mains electricity supplies tend to supply power at a voltage of 100 V or more, and accidental contact with such high voltages can be harmful.

It could be desirable to have a portable medical device in which the power supply is incorporated within the handpiece itself, however, depending on the power required this could be problematic. Additionally, any portable power supply should be able to produce power over a sufficiently long period of time that the procedure the practitioner is using the device in can be completed. Portable power supplies, such as batteries, tend to be heavy and generally the longer the device is needed for and the more power that is needed the heavier the required power supply will be. Therefore handpieces requiring a substantial amount of power during operation necessitate a heavy power supply. As described previously, it is desirable that the handpiece of a medical device be lightweight, therefore incorporating a heavy power supply within a handpiece is not desirable. Additionally, the handpiece may come into contact with bodily fluids during use and therefore will commonly require sterilisation following each procedure it is used in. Sterilisation usually involves the handpiece being placed in an autoclave at an elevated temperature (e.g. 134 °C) for a minimum period of time (e.g. 18 mins). Providing a power supply capable of reuse following sterilisation is difficult. Equally isolating a power supply within the handpiece such that it is not exposed to bodily fluids is also difficult.

In one embodiment of the present invention, a convenient means to provide power to a medical device comprises a handpiece that requires electrical power during operation may comprise an integration unit, the handpiece being connected to the integration unit and the integration unit comprising a docking station for at least one battery and means to monitor the operation of the device.

Isolating the medical device from the mains electricity supply is advantageous as it prevents accidental exposure of users of the device and patients to high voltages. Additionally, mains electricity is earthed whereas the at least one battery used in the present invention is not. This provides an additional safety feature.

The at least one battery may be disposable or may be rechargeable. If a rechargeable battery is used, a recharger will be required. The recharger may be connectable to the mains power supply to readily enable the battery to be recharged. Generally, the recharger would not be used in a sterile area.

A range of rechargeable batteries are suitable for use in the present invention, including nickel metal hydride (NiMH), nickel cadmium (NiCd), lithium ion (Li-ion) and lithium polymer (Li-polymer) batteries.

The docking station enables the power supplied from the at least one battery to be supplied to the handpiece. The integration unit includes means of connecting the supply of a resource required by the handpiece to the handpiece. This encompasses the supply of power, but may also encompass the supply of suction, fluid(s) and/or pharmaceutical products.

Additionally, the integration unit includes means to monitor the operation of the device. The means to monitor the operation of the device may include one or more of means to monitor the electrical power used by the device, means to monitor the supply of fluid(s) to the device, means to monitor the evacuation of fluid(s) from the device, means to monitor the supply of pharmaceutical products to the device, ultrasound monitoring means and suction flow monitoring means.

Suction may be supplied to the medical device via the integration unit or independently of the integration unit, e.g. from a vacuum system within the hospital in which the device is being used or by a separate suction pump.

The integration unit may include control means to control the operation of the handpiece, or the control means may be integrated into the handpiece itself, or control means may be integrated into both the integration unit and the handpiece.

Conveniently, the integration unit may comprise fixing means to ensure that a practitioner can manipulate the medical device freely during use without any movement of the integration unit interfering with the procedure. The fixing means may be suitable to enable the integration unit to be fixed to an operating table. The fixing means may comprise a clamp and or suction pads.

In accordance with a second aspect of the present disclosure use of the medical device of the present invention is provided.

Use of the medical system of the present invention may include use of an endoscope, optionally an endoscope comprising irrigation means. The irrigation means may simplify use of the endoscope and may reduce the temperature of the probe. Saline is commonly used as the irrigation fluid.

### Example:

In order that the invention may be more fully understood, the following Figure and Example are provided by way of illustration only.

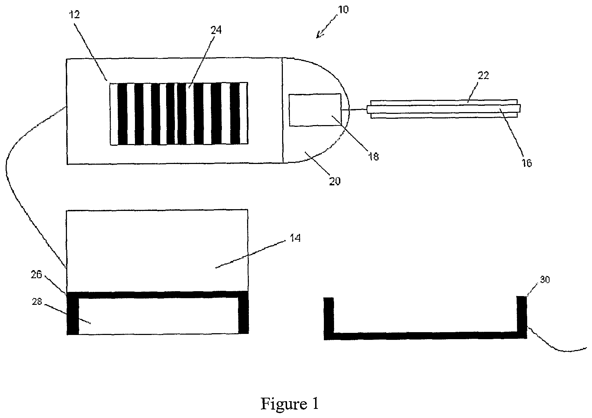

Figure 1 is a schematic diagram of one embodiment of a medical device according to the present invention.

Figure 1 shows a portable lithotripsy device 10 comprising a handpiece 12 and an integration unit 14. A probe 16 is fixed to the handpiece 12 using a chuck 18 mounted within the end cap 20 of the handpiece. In use, the probe 16 is fed through an endoscope 22 into a patient. The handpiece 12 comprises a piezo stack 24 for generating ultrasonic vibrations that are transmitted by the probe 16 to a urinary tract stone within the patient. Suction may be supplied to the probe 16 using suction means (not shown) that is connected to the handpiece 12 via the integration unit 14 or directly to the handpiece 12. Power is supplied to the handpiece 12 by the battery 26 connected to the docking station 28 of the integration unit 14. The battery 26 may be disconnected from the docking station 28 for recharging using a recharger 30, which, in use, is connected to the mains supply of electricity.

### Example:

A system according to the present invention was tested in an endoscope with the piezoelectric motor set to maximum power (60 W) and the irrigation system set to a minimum flow rate. After 5 minutes of continuous operation the temperature of the handpiece and the tip of the probe was measured and found to be 45 °C and 25 °C respectively.

## Claims

1. A medical device comprising a hollow probe (16) and ultrasound generation means (24) disposed to direct a generated ultrasound wave to the tip of the hollow probe, wherein:
the device further comprises suction monitoring means that comprises a flow sensor, wherein, in use, suction is applied through the hollow probe:
the ultrasound generation means is adjusted in response to a suction flow rate through the probe detected by the suction monitoring means such that a decrease in the monitored suction flow rate through the hollow probe causes an increase in the magnitude and/or frequency of the ultrasound wave generated by the ultrasound generation means and directed to the tip of the probe:
the device additionally comprises a temperature monitoring means, and wherein the ultrasound generation means is adjusted in response to a temperature detected by temperature monitoring means, and wherein the temperature monitoring means comprises at least one temperature sensor; and
the at least one temperature sensor is coupled to the hollow probe, or the at least one temperature sensor is located within the device, or at least two temperature sensors are used one being coupled to the hollow probe and one being located within the device, the temperature sensor(s) being adapted to measure the temperature of the fluid flowing through the device.

2. The medical device according to claim 1, wherein the suction monitoring means further comprises pressure sensors.

3. The medical device according to any preceding claim, wherein an audible alarm, a visible alarm or both an audible alarm and a visible alarm is produced in response to the suction flow rate detected by the suction monitoring means.

4. The medical device according to any preceding claim, wherein an electronic signal is produced in response to the suction flow rate detected by the suction monitoring means.

5. The medical device according to any preceding claim, wherein the hollow probe is reusable.

6. The medical device according to any one of claims 1 to 4, wherein the hollow probe is disposable.

7. The medical device according to any preceding claim, wherein the hollow probe has an external diameter of from 3 mm to 6 mm.

8. The medical device according to any preceding claim, wherein the hollow probe has an internal diameter of from 2 mm to 5 mm.

9. The medical device according to any preceding claim, wherein the hollow probe has a length of from 200 mm to 400 mm.

10. The medical device according to any preceding claim, wherein the hollow probe comprises at least one metal selected from the list consisting of titanium and stainless steel.

11. The medical device according to any preceding claim, wherein the ultrasound generation means comprises a piezoelectric motor.

12. The medical device according to any preceding claim, wherein the ultrasound generation means may be adjusted to alter the strength and/or the frequency of the ultrasound.

13. The medical device according to any preceding claim, wherein the device additionally comprises at least one chuck for reversibly fixing the probe to a body of the device.

14. The medical device according to claim 13, wherein the at least one chuck is located within an end cap located on the distal end of the body of the device.

15. The medical device according to claim 13, wherein the probe passes through the body of the device from the distal end of the body of the device to the proximal end of the body of the device and the at least one chuck is located within an end cap located on the proximal end of the body of the device.

16. The medical device according to claim 13, wherein the at least one chuck is located within an end cap located on the distal end of the body of the device or the least one chuck is located within an end cap located on the proximal end of the device.

17. The medical device according to any of claims 13 to 16, wherein the at least one chuck comprises metal.

18. The medical device according to any preceding claim, wherein the device additionally comprises an integration unit, the body of the device being connected to the integration unit and the integration unit comprising a docking station for at least one battery and means to monitor the operation of the device.

19. The medical device according to claim 18, wherein the at least one battery is rechargeable.

20. The medical device according to claim 19, wherein the at least one rechargeable battery is selected from the list consisting of nickel metal hydride, nickel cadmium, lithium ion and lithium polymer batteries.

21. The medical device according to any of claims 18 to 20, wherein the integration unit comprises control means.

22. The medical device according to any preceding claim, comprising a handpiece wherein the handpiece of the device comprises control means.

## Patentansprüche

1. Medizinische Vorrichtung, umfassend eine hohle Sonde (16) und ein Ultraschallerzeugungsmittel (24), das zum Leiten einer erzeugten Ultraschallwelle zur Spitze der hohlen Sonde angeordnet ist, wobei:
die Vorrichtung ferner ein Saugüberwachungsmittel umfasst, das einen Durchflusssensor umfasst, wobei im Gebrauch Absaugung durch die hohle Sonde aufgebracht wird:
das Ultraschallerzeugungsmittel als Reaktion auf eine vom Saugüberwachungsmittel nachgewiesene Saugströmungsrate durch die Sonde angepasst wird, derart, dass eine Verringerung der überwachten Saugströmungsrate durch die hohle Sonde eine Erhöhung der Größe und/oder Frequenz der von dem Ultraschallerzeugungsmittel erzeugten und zur Spitze der Sonde geleiteten Ultraschallwelle verursacht:
die Vorrichtung zusätzlich ein Temperaturüberwachungsmittel umfasst, und wobei das Ultraschallerzeugungsmittel als Reaktion auf eine vom Temperaturüberwachungsmittel nachgewiesene Temperatur angepasst wird, und wobei das Temperaturüberwachungsmittel zumindest einen Temperatursensor umfasst; und
der zumindest eine Temperatursensor mit der hohlen Sonde verbunden ist, oder der zumindest eine Temperatursensor in der Vorrichtung angeordnet ist, oder wenigstens zwei Temperatursensoren verwendet werden, wobei einer mit der hohlen Sonde verbunden ist und der andere in der Vorrichtung angeordnet ist, wobei der (die) Temperatursensor(en) dazu ausgelegt ist (sind), die Temperatur der durch die Vorrichtung fließenden Flüssigkeit zu messen.

2. Medizinische Vorrichtung nach Anspruch 1, wobei das Saugüberwachungsmittel ferner Drucksensoren umfasst.

3. Medizinische Vorrichtung nach einem der vorhergehenden Ansprüche, wobei ein akustischer Alarm, ein optischer Alarm oder sowohl ein akustischer Alarm als auch ein optischer Alarm als Reaktion auf die vom Saugüberwachungsmittel nachgewiesene Saugströmungsrate erzeugt wird.

4. Medizinische Vorrichtung nach einem der vorhergehenden Ansprüche, wobei ein elektronisches Signal als Reaktion auf die vom Saugüberwachungsmittel nachgewiesene Saugströmungsrate erzeugt wird.

5. Medizinische Vorrichtung nach einem der vorhergehenden Ansprüche, wobei die hohle Sonde wiederverwendbar ist.

6. Medizinische Vorrichtung nach einem der Ansprüche 1 bis 4, wobei die hohle Sonde ein Einwegartikel ist.

7. Medizinische Vorrichtung nach einem der vorhergehenden Ansprüche, wobei die hohle Sonde einen Außendurchmesser von 3 mm bis 6 mm aufweist.

8. Medizinische Vorrichtung nach einem der vorhergehenden Ansprüche, wobei die hohle Sonde einen Innendurchmesser von 2 mm bis 5 mm aufweist.

9. Medizinische Vorrichtung nach einem der vorhergehenden Ansprüche, wobei die hohle Sonde eine Länge von 200 mm bis 400 mm aufweist.

10. Medizinische Vorrichtung nach einem der vorhergehenden Ansprüche, wobei die hohle Sonde zumindest ein aus der aus Titan und Edelstahl bestehenden Liste ausgewähltes Metall umfasst.

11. Medizinische Vorrichtung nach einem der vorhergehenden Ansprüche, wobei das Ultraschallerzeugungsmittel einen piezoelektrischen Motor umfasst.

12. Medizinische Vorrichtung nach einem der vorhergehenden Ansprüche, wobei das Ultraschallerzeugungsmittel zur Änderung der Stärke und/oder Frequenz des Ultraschalls angepasst werden kann.

13. Medizinische Vorrichtung nach einem der vorhergehenden Ansprüche, wobei die Vorrichtung zusätzlich wenigstens ein Spannfutter zur reversiblen Fixierung der Sonde an einem Gehäuse der Vorrichtung umfasst.

14. Medizinische Vorrichtung nach Anspruch 13, wobei das wenigstens eine Spannfutter in einer Endkappe angeordnet ist, die auf dem distalen Ende des Gehäuses der Vorrichtung angeordnet ist.

15. Medizinische Vorrichtung nach Anspruch 13, wobei die Sonde durch das Gehäuse der Vorrichtung vom distalen Ende des Gehäuses der Vorrichtung zum proximalen Ende des Gehäuses der Vorrichtung verläuft und das wenigstens eine Spannfutter in einer Endkappe angeordnet ist, die auf dem proximalen Ende des Gehäuses der Vorrichtung angeordnet ist.

16. Medizinische Vorrichtung nach Anspruch 13, wobei das wenigstens eine Spannfutter in einer Endkappe angeordnet ist, die auf dem distalen Ende des Gehäuses der Vorrichtung angeordnet ist, und das wenigstens eine Spannfutter in einer Endkappe angeordnet ist, die auf dem proximalen Ende der Vorrichtung angeordnet ist.

17. Medizinische Vorrichtung nach einem der Ansprüche 13 bis 16, wobei das wenigstens eine Spannfutter Metall umfasst.

18. Medizinische Vorrichtung nach einem der vorhergehenden Ansprüche, wobei die Vorrichtung zusätzlich eine Integrationseinheit umfasst, wobei das Gehäuse der Vorrichtung mit der Integrationseinheit verbunden ist und die Integrationseinheit eine Andockstation für wenigstens eine Batterie und ein Mittel zur Überwachung des Betriebs der Vorrichtung umfasst.

19. Medizinische Vorrichtung nach Anspruch 18, wobei die wenigstens eine Batterie aufladbar ist.

20. Medizinische Vorrichtung nach Anspruch 19, wobei die wenigstens eine aufladbare Batterie aus der aus Nickel-Metallhydrid-, Nickel-Cadmium-, Lithium-Ionen- und Lithium-Polymer-Batterien bestehenden Liste ausgewählt ist.

21. Medizinische Vorrichtung nach einem der Ansprüche 18 bis 20, wobei die Integrationseinheit Steuermittel umfasst.

22. Medizinische Vorrichtung nach einem der vorhergehenden Ansprüche, umfassend ein Handstück, wobei das Handstück der Vorrichtung Steuermittel umfasst.

## Revendications

1. Dispositif médical comportant une sonde (16) creuse et un moyen (24) de génération d'ultrasons disposé pour diriger une onde ultrasonore générée vers le bout de la sonde creuse,
dans lequel :
le dispositif comporte en outre un moyen de surveillance d'une aspiration qui comporte un capteur de débit, dans lequel, lors de l'utilisation, une aspiration est appliquée dans la sonde creuse :
le moyen de génération d'ultrasons est ajusté en réponse à un débit d'aspiration dans la sonde détecté par le moyen de surveillance de l'aspiration de sorte qu'une diminution du débit d'aspiration surveillé dans la sonde creuse entraîne une augmentation de l'ampleur et/ou la fréquence de l'onde ultrasonore générée par le moyen de génération d'ultrasons et dirigée vers le bout de la sonde :
le dispositif comporte additionnellement un moyen de surveillance de la température, et dans lequel le moyen de génération d'ultrasons est ajusté en réponse à une température détectée par le moyen de surveillance de la température, et dans lequel le moyen de surveillance de la température comporte au moins un capteur de température ; et
ledit au moins un capteur de température est accouplé à la sonde creuse, ou ledit au moins un capteur de température est situé à l'intérieur du dispositif, ou au moins deux capteurs de température sont utilisés, l'un étant accouplé à la sonde creuse et l'autre étant situé à l'intérieur du dispositif, le ou les capteurs de température étant conçus pour mesurer la température du fluide s'écoulant dans le dispositif.

2. Dispositif médical selon la revendication 1, dans lequel le moyen de surveillance de l'aspiration comporte en outre des capteurs de pression.

3. Dispositif médical selon l'une quelconque des revendications précédentes, dans lequel une alarme sonore, une alarme visuelle ou les deux (sonore et visuelle) sont produites en réponse au débit d'aspiration détecté par le moyen de surveillance de l'aspiration.

4. Dispositif médical selon l'une quelconque des revendications précédentes, dans lequel un signal électronique est produit en réponse au débit d'aspiration détecté par le moyen de surveillance de l'aspiration.

5. Dispositif médical selon l'une quelconque des revendications précédentes, dans lequel la sonde creuse est réutilisable.

6. Dispositif médical selon l'une quelconque des revendications 1 à 4, dans lequel la sonde creuse est jetable.

7. Dispositif médical selon l'une quelconque des revendications précédentes, dans lequel la sonde creuse a un diamètre extérieur compris entre 3 et 6 mm.

8. Dispositif médical selon l'une quelconque des revendications précédentes, dans lequel la sonde creuse a un diamètre intérieur compris entre 2 et 5 mm.

9. Dispositif médical selon l'une quelconque des revendications précédentes, dans lequel la sonde creuse a une longueur comprise entre 200 et 400 mm.

10. Dispositif médical selon l'une quelconque des revendications précédentes, dans lequel la sonde creuse comporte au moins un métal sélectionné dans la liste constituée de titane et d'acier inoxydable.

11. Dispositif médical selon l'une quelconque des revendications précédentes, dans lequel le moyen de génération d'ultrasons comporte un moteur piézo-électrique.

12. Dispositif médical selon l'une quelconque des revendications précédentes, dans lequel le moyen de génération d'ultrasons peut être ajusté pour modifier la force et/ou la fréquence de l'ultrason.

13. Dispositif médical selon l'une quelconque des revendications précédentes, dans lequel le dispositif comporte additionnellement au moins un mandrin permettant de fixer de manière réversible la sonde à un corps du dispositif.

14. Dispositif médical selon la revendication 13, dans lequel ledit au moins un mandrin est situé à l'intérieur d'un bouchon d'extrémité situé sur l'extrémité distale du corps du dispositif.

15. Dispositif médical selon la revendication 13, dans lequel la sonde traverse le corps du dispositif de l'extrémité distale du corps du dispositif à l'extrémité proximale du corps du dispositif et ledit au moins un mandrin est situé à l'intérieur d'un bouchon d'extrémité situé sur l'extrémité proximale du corps du dispositif.

16. Dispositif médical selon la revendication 13, dans lequel ledit au moins un mandrin est situé à l'intérieur d'un bouchon d'extrémité situé sur l'extrémité distale du corps du dispositif ou ledit au moins un mandrin est situé à l'intérieur d'un bouchon d'extrémité situé sur l'extrémité proximale du dispositif.

17. Dispositif médical selon l'une quelconque des revendications 13 à 16, dans lequel ledit au moins un mandrin est constitué de métal.

18. Dispositif médical selon l'une quelconque des revendications précédentes, dans lequel le dispositif comporte additionnellement une unité d'intégration, le corps du dispositif étant relié à l'unité d'intégration et l'unité d'intégration comportant une station d'accueil pour au moins un accumulateur et un moyen de surveillance du fonctionnement du dispositif.

19. Dispositif médical selon la revendication 18, dans lequel ledit au moins un accumulateur est rechargeable.

20. Dispositif médical selon la revendication 19, dans lequel ledit au moins un accumulateur rechargeable est sélectionné dans la liste constituée d'accumulateurs à hydrure métallique de nickel, au nickel-cadmium, au lithium-ion et au lithium polymère.

21. Dispositif médical selon l'une quelconque des revendications 18 à 20, dans lequel l'unité d'intégration comporte un moyen de commande.

22. Dispositif médical selon l'une quelconque des revendications précédentes, comportant une pièce à main, dans lequel la pièce à main du dispositif comporte un moyen de commande.
